# EUROPÄISCHE PATENTSCHRIFT

(11) **EP 4 196 177 B1**
(45) Veröffentlichungstag und Bekanntmachung des Hinweises auf die Patenterteilung: **17.04.2024**
(21) Anmeldenummer: 21754937.7
(22) Anmeldetag: 23.07.2021
(51) Int. Cl.: A61L 2/10, B60Q 3/208

(54) **DESINFEKTIONSSYSTEM**
DISINFECTION SYSTEM
SYSTÈME DE DÉSINFECTION

(30) Priorität: 11.08.2020 DE 102020004883
(43) Veröffentlichungstag der Anmeldung: 21.06.2023
(73) Patentinhaber: Mercedes-Benz Group AG, 70372 Stuttgart (DE)
(72) Erfinder: SALKIC, Asmir, 89077 Ulm (DE)
(74) Vertreter: Novagraaf Group
(86) Internationale Anmeldenummer: PCT/EP2021/070702
(87) Internationale Veröffentlichungsnummer: WO 2022/033840

(56) Entgegenhaltungen:
- WO-A1-2019/139743
- DE-A1-102018 002 328
- US-A1- 2020 061 223
- US-A1- 2020 306 398
- US-B2- 9 492 575

## Beschreibung

Die Erfindung betrifft ein Desinfektionssystem für ein Fahrzeug nach dem Oberbegriff des Anspruchs 1. Die Erfindung betrifft auch ein Verfahren zum Steuern des Desinfektionssystems. Die Erfindung betrifft zudem ein Fahrzeug mit dem Desinfektionssystem.

Konventionelle Fahrzeuge weisen keine Desinfektionssysteme auf, die eine automatische und schnelle Desinfektion ermöglichen. Aus diesem Grund werden die Fahrzeuge bei Bedarf manuell und aufwändig mit Chemikalien desinfiziert. Insbesondere bei Leih-Fahrzeugen besteht der Bedarf einer automatischen und schnellen Desinfektion, um die Übertragung von Viren und Bakterien von einem Fahrgast zum anderen Fahrgast zu unterbinden.

Es ist bekannt, dass elektromagnetische Strahlung im UV-Bereich desinfizierend wirkt. Wenn Viren, Bakterien oder Protozoen der UV-Strahlung im Wellenlängenbereich 200-300 nm ausgesetzt sind, verlieren sie die Reproduktions- und Infektionsfähigkeit. Die UV-Strahlung wird immer häufiger zur umweltfreundlichen, chemikalienfreien und hochwirksamen Desinfektion verwendet. Nachteiligerweise ist die UV-Strahlung im Wellenlängenbereich 200-300 nm auch für die menschliche Haut und das menschliche Auge potenziell schädlich.

In der DE 10 2017 115 060 A1 wird vorgeschlagen, in einem Fahrzeug eine Lichtquelle zum Erzeugen von elektromagnetischer Strahlung im UV-B-Bereich einzusetzen. Dadurch können die Vitamin-D-Produktion bei Fahrgästen intensiviert und Keime - also Viren und Bakterien - abgetötet werden. Nachteiligerweise weist die Strahlung im UV-B-Bereich eine schwache desinfizierende Wirkung auf.

US 9 492 575 B2 offenbart ein Verfahren zum Steuern eines Beleuchtungssystems eines Fahrzeugs. Dabei kann die in mehreren Schichten erzeugte nicht umgewandelte UV-Strahlung zur Desinfektion genutzt werden. Das Desinfizieren wird in Abwesenheit der Fahrgäste durchgeführt.

WO 2019/139743 A1 und die D3 US 2020/061223 A1 offenbaren jeweils ein Verfahren, bei dem eine UV-Lichtquelle zur Desinfektion des Fahrzeugs eingesetzt wird. Das Desinfizieren wird dabei zweckgemäß in Abwesenheit der Fahrgäste durchgeführt.

DE 10 2018 002328 A1 offenbart ein Verfahren, bei dem das Fahrzeug mittels UV-C-Strahlung in Abwesenheit der Fahrgäste desinfiziert wird. Zudem kann auch sichtbare UV-A-Strahlung (Schwarzlicht) oder sichtbares blaues Licht emittiert werden, um dem Fahrgast das Gefühl der durchgeführten Desinfektion zu vermitteln.

Die Aufgabe der Erfindung ist es daher, für ein Desinfektionssystem der gattungsgemäßen Art eine verbesserte oder zumindest alternative Ausführungsform anzugeben, bei der die beschriebenen Nachteile überwunden werden. Die Aufgabe der Erfindung ist es auch, ein Verfahren zum Steuern des Desinfektionssystems bereitzustellen. Die Aufgabe der Erfindung ist es weiter, ein Kraftfahrzeug mit dem Desinfektionssystem bereitzustellen.

Diese Aufgaben werden erfindungsgemäß durch den Gegenstand der unabhängigen Ansprüche gelöst. Vorteilhafte Ausführungsformen sind Gegenstand der abhängigen Ansprüche.

Das Desinfektionssystem ist für ein Fahrzeug vorgesehen. Das Desinfektionssystem weist dabei eine erste Lichtquelle für einen Innenraum des Fahrzeugs auf, die zum Erzeugen von Strahlung im UV-Bereich ausgelegt ist. Erfindungsgemäß ist die erste Lichtquelle zum Erzeugen von Strahlung im UV-B- und/oder UV-C-Bereich ausgelegt. Zudem weist das Desinfektionssystem zusätzlich eine zweite Lichtquelle auf, die zum Erzeugen von Strahlung im UV-C-Bereich ausgelegt ist.

In der vorliegenden Erfindung ist unter dem Begriff "Strahlung" stets die elektromagnetische Strahlung bzw. Licht zu verstehen. Die Abkürzung "UV" steht für den Begriff "Ultraviolett". Der angegebene UV-B-Bereich und der angegebene UV-C-Bereich beziehen sich auf Wellenlängen der elektromagnetischen Strahlung im UV-Bereich. Nach der üblichen Definition liegen die Wellenlängen der elektromagnetischen Strahlung im UV-B-Bereich bei 315-280 nm und die Wellenlängen der Strahlung im UV-C-Bereich bei 280-100 nm. Die hier und weiter verwendete Einheit "nm" steht für Nanometer.

Dabei ist die erste Lichtquelle zum Erzeugen von Strahlung im Bereich 100-300 nm ausgebildet. Vorzugsweise ist die erste Lichtquelle zum Erzeugen von Strahlung im Bereich 200-300 nm ausgebildet. Die zweite Lichtquelle ist zum Erzeugen von Strahlung im Bereich 210-230 nm ausgebildet. Vorzugsweise ist die zweite Lichtquelle zum Erzeugen von Strahlung im Bereich 222 nm ausgebildet.

Die erste Lichtquelle strahlt in einem breiten UV-Bereich und kann zur Desinfektion des leeren Fahrzeugs bzw. des Fahrzeugs ohne Fahrgäste eingesetzt werden. Die zweite Lichtquelle strahlt in einem engen UV-Bereich und daher schonend für die menschliche Haut und das menschliche Auge. Die zweite Lichtquelle kann daher zur Desinfektion des besetzten Fahrzeugs bzw. des Fahrzeugs mit Fahrgästen eingesetzt werden. Mit dem erfindungsgemäßen Desinfektionssystem kann der Innenraum des Fahrzeugs berührungslos, chemikalienfrei und wirksam desinfiziert werden. Die Desinfektion kann vorteilhafterweise auch in einem besetzten Fahrzeug bzw. im Fahrzeug mit Fahrgästen hochwirksam erfolgen.

Vorteilhafterweise kann vorgesehen sein, dass die zweite Lichtquelle zum Erzeugen von Strahlung mit einer Strahlenkonzentration von wenigstens 12 mJ/cm² ausgebildet ist. Dadurch kann eine Regenerierung von Viren und Bakterien effizient verhindert werden. Dabei kann die genaue Strahlenkonzentration der Strahlung basierend auf Feldtests bzw. auf Bioassay-Validierung bestimmt werden. Die hier und weiter verwendete Einheit "mJ" steht für Millijoule. Die verwendete Einheit "cm" steht hier und weiter für Zentimeter.

Erfindungsgemäß weist das Desinfektionssystem eine Steuerung auf, die die erste Lichtquelle und die zweite Lichtquelle steuert. Die Steuerung ist zum Ausführen des Verfahrens zum Steuern des Desinfektionssystems ausgebildet.

Die Steuerung des Desinfektionssystems kann beispielweise ein Teil einer künstlichen Intelligenz (KI) sein. Insbesondere kann die Steuerung die beiden Lichtquellen ein- und ausschalten. Das Ein- und Ausschalten der jeweiligen Lichtquelle kann dabei davon abhängig erfolgen, ob die Desinfektion des leeren Fahrzeugs bzw. des Fahrzeugs ohne Fahrgäste oder des besetzten Fahrzeugs bzw. des Fahrzeugs mit Fahrgästen erfolgen soll. Erfindungsgemäß weist das Desinfektionssystem wenigstens eine Identifikationseinheit zum Identifizieren von Fahrgästen in dem Fahrzeug.

Vorzugsweise ist die Identifikationseinheit eine Kamera oder ein Radar.

Vorteilhafterweise können die erste Lichtquelle und/oder die zweite Lichtquelle einteilig oder mehrteilig sein. Mit anderen Worten können die erste Lichtquelle und/oder die zweite Lichtquelle jeweils aus einer Leuchteinheit oder aus mehreren Leuchteinheiten bestehen. Vorteilhafterweise können die erste Lichtquelle und/oder die zweite Lichtquelle mehrteilig sein und in dem Innenraum des Fahrzeugs verteilt angeordnet sein. Die Anordnung der einzelnen Leuchteinheiten in dem Innenraum des Fahrzeugs kann dabei so erfolgen, dass der gesamte Innenraum des Fahrzeugs von der ersten Lichtwelle und von der zweiten Lichtquelle bestrahlt werden kann.

Zusammenfassend kann durch das erfindungsgemäße Desinfektionssystem eine berührungslose und sichere Desinfektion des Innenraums des Fahrzeugs erfolgen. Die Desinfektion kann dabei auf eine umweltfreundliche, chemikalienfreie, nachhaltige und hochwirksame Weise erfolgen. Bei dem erfindungsgemäßen Desinfektionssystem entfallen die Lagerung, der Transport und die Handhabung von giftigen Chemikalien und entstehen keine chemischen und insbesondere krebsfördernden Nebenprodukte. Für das Fahrzeug besteht zudem keine Korrosionsgefahr.

Die Erfindung betrifft auch ein Verfahren zum Steuern eines oben beschriebenen Desinfektionssystems. Bei dem Verfahren bestimmt zuerst eine Steuerung des Desinfektionssystems mittels wenigstens einer Identifikationseinheit, ob sich Fahrgäste in dem Fahrzeug befinden. Die Identifikationseinheit ist vorzugsweise eine Kamera oder ein Radar. Abhängig davon, ob sich Fahrgäste in dem Fahrzeug befinden, schaltet die Steuerung die erste Lichtquelle oder die zweite Lichtquelle ein.

Erfindungsgemäß ist vorgesehen, dass, wenn sich keine Fahrgäste in dem Fahrzeug befinden, die Steuerung die erste Lichtquelle für wenigstens 6 Sekunden einschaltet. Wie oben beschrieben, ist die erste Lichtquelle zum Erzeugen von Strahlung im Bereich 100-300 nm ausgebildet. Vorzugsweise ist die erste Lichtquelle zum Erzeugen von Strahlung im Bereich 200-300 nm ausgebildet. Mit der ersten Lichtquelle kann ein sicheres und nahezu vollständiges Abtöten von Viren und Bakterien bewirkt werden. Da die Strahlung im Bereich 200-300 nm für menschliche Haut und das menschliche Auge schädlich sein kann, wird die erste Lichtquelle eingeschaltet, wenn keine Fahrgäste sich in dem Fahrzeug befinden. Dadurch kann eine Verletzung der Fahrgäste verhindert werden.

Erfindungsgemäß ist zudem vorgesehen, dass, wenn sich Fahrgäste in dem Fahrzeug befinden, die Steuerung die zweite Lichtquelle für wenigstens 2,5 Sekunden einschaltet. Wie oben beschrieben, ist die zweite Lichtquelle zum Erzeugen von Strahlung im Bereich 210-230 nm ausgebildet. Vorzugsweise ist die zweite Lichtquelle zum Erzeugen von Strahlung im Bereich 222 nm vorgesehen. Die Strahlung im Bereich 222 nm ist für die menschliche Haut und das menschliche Auge weniger schädlich als die Strahlung im breiten Bereich 200-300 nm. Die meisten Viren und Bakterien werden dennoch abgetötet.

Mit dem erfindungsgemäßen Verfahren kann die Desinfektion im leeren Fahrzeug bzw. im Fahrzeug ohne Fahrgäste und im besetzten Fahrzeug bzw. im Fahrzeug mit Fahrgästen erfolgen. Dadurch kann die Desinfektion zu einem beliebigen Zeitpunkt erfolgen, und die Fahrgäste können vor Viren und Bakterien besser geschützt werden. Das Desinfektionssystem kann dabei automatisch betrieben werden. Zudem kann das Desinfektionssystem energetisch effizient betrieben bzw. kann der maximale Desinfektionseffekt bei dem minimalen Energieverbrauch erreicht werden.

Die Erfindung betrifft auch ein Fahrzeug mit einem oben beschriebenen Desinfektionssystem. Die erste Lichtquelle und die zweite Lichtquelle sind dabei in einem Innenraum des Fahrzeugs an einem Dach des Fahrzeugs und/oder an einer tragenden Säule des Fahrzeugs und/oder an einer Konsole des Fahrzeugs befestigt. Vorteilhafterweise können die erste Lichtquelle und die zweite Lichtquelle so nebeneinander angeordnet sein, dass die Strahlungsausbreitung der ersten Lichtquelle und die Strahlungsausbreitung der zweiten Lichtquelle in dem Innenraum des Fahrzeugs identisch sind. Dadurch werden alle Bereiche des Innenraums des Fahrzeugs mit der ersten Lichtquelle und mit der zweiten Lichtquelle bestrahlt. Dadurch kann die Desinfektion des Innenraums mit der ersten Lichtquelle und mit der zweiten Lichtquelle auf gleiche Weise vollständig und effizient erfolgen.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.

Dabei zeigen:
- Fig. 1: eine schematische Ansicht eines Fahrzeugs mit einem erfindungsgemäßen Desinfektionssystem;
- Fig. 2: ein Steuerschema eines erfindungsgemäßen Verfahrens zum Steuern des Desinfektionssystems.

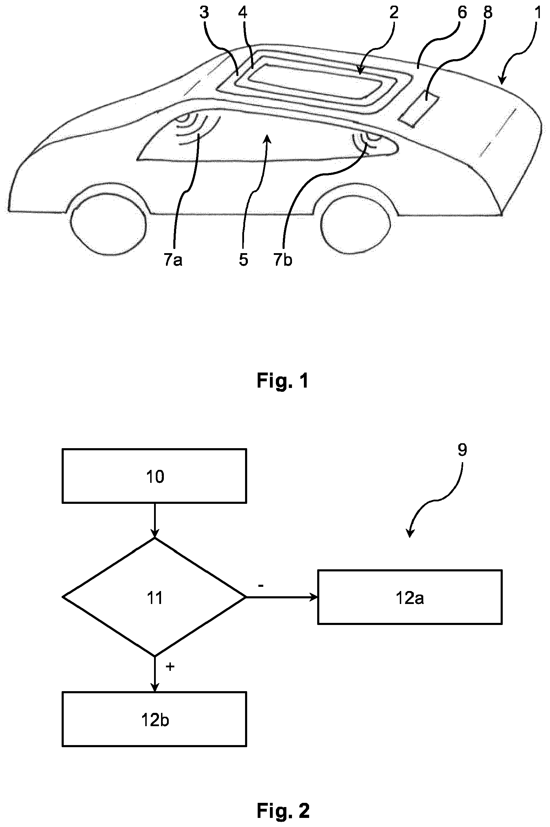

Fig. 1 zeigt eine schematische Ansicht eines Fahrzeugs 1 mit einem erfindungsgemäßen Desinfektionssystem 2. Das Desinfektionssystem 2 weist dabei eine erste Lichtquelle 3 und eine zweite Lichtquelle 4 auf, die in einem Innenraum 5 des Fahrzeugs 1 an einem Dach 6 befestigt sind. Die erste Lichtquelle 3 und die zweite Lichtquelle 4 sind dabei nebeneinander angeordnet, so dass die Strahlungsausbreitung der ersten Lichtquelle 3 und die Strahlungsausbreitung der zweiten Lichtquelle 4 in dem Innenraum 5 identisch sind.

Die erste Lichtquelle 3 ist zum Erzeugen von Strahlung im UV-B- und/oder UV-C-Bereich ausgebildet. Vorteilhafterweise kann die erste Lichtquelle 3 zum Erzeugen von Strahlung im Bereich 100-300 nm ausgebildet sein. Vorzugsweise ist die erste Lichtquelle 3 zum Erzeugen von Strahlung im Bereich 200-300 nm ausgebildet. Die erste Lichtquelle 3 strahlt in einem breiten UV-Bereich und kann zur Desinfektion des leeren Fahrzeugs 1 bzw. des Fahrzeugs 1 ohne Fahrgäste eingesetzt werden.

Die zweite Lichtquelle 4 ist zum Erzeugen von Strahlung im UV-C-Bereich ausgebildet. Vorteilhafterweise kann die zweite Lichtquelle 4 zum Erzeugen von Strahlung im Bereich 210-230 nm ausgebildet sein. Vorzugsweise ist die zweite Lichtquelle 4 zum Erzeugen von Strahlung im Bereich 222 nm ausgebildet. Die zweite Lichtquelle 4 strahlt in einem engen UV-Bereich und kann zur Desinfektion des besetzten Fahrzeugs 1 bzw. des Fahrzeugs 1 mit Fahrgästen eingesetzt werden. Die zweite Lichtquelle 4 ist zweckgemäß zum Erzeugen von Strahlung mit einer Strahlenkonzentration von wenigstens 12 mJ/cm² ausgebildet, um ein sicheres Desinfizieren des Innenraums 5 zu ermöglichen.

Das Desinfektionssystem 2 weist zudem zwei Identifikationseinheiten 7a und 7b auf, wobei die Identifikationseinheiten 7a und 7b hier als Kameras/Radare ausgebildet sind. Durch die Identifikationseinheiten 7a und 7b kann das Desinfektionssystem 2 feststellen, ob sich Fahrgäste in dem Innenraum 5 befinden oder nicht befinden. Basierend darauf kann dann eine Steuerung 8 des Desinfektionssystems 2 die Lichtquellen 3 und 4 steuern.

Das Steuern des Desinfektionssystems 2 erfolgt in einem erfindungsgemäßen Verfahren 9. Fig. 2 zeigt dabei ein Steuerschema des Verfahrens 9. Nach einem Startschritt 10 bestimmt die Steuerung 8 in einem Identifikationsschritt 11 mittels der Identifikationseinheiten 7a und 7b, ob sich Fahrgäste in dem Innenraum 5 des Fahrzeugs 1 befinden bzw. aufhalten.

Befinden sich in dem Innenraum 5 des Fahrzeugs 1 keine Fahrgäste - in Fig. 2 mit "-" gekennzeichnet, - so wird ein Desinfektionsschritt 12a durchgeführt. Dabei wird zur Desinfektion des Innenraums 5 die erste Lichtquelle 3 für wenigstens 6 Sekunden eingeschaltet und eine gründliche Desinfektion des Innenraums 5 vorgenommen. Befinden sich in dem Innenraum 5 Fahrgäste - in Fig. 2 mit "+" gekennzeichnet, - so wird ein Desinfektionsschritt 12b durchgeführt. Dabei wird zur Desinfektion des Innenraums 5 die zweite Lichtquelle 4 für wenigstens 2,5 Sekunden eingeschaltet und eine für die menschliche Haut und das menschliche Auge schonende Desinfektion des Innenraums 5 vorgenommen

## Patentansprüche

1. Verfahren (9) zum Steuern eines Desinfektionssystems (2) eines Fahrzeugs (1),
- wobei das Desinfektionssystem (2) eine erste Lichtquelle (3) für einen Innenraum (5) des Fahrzeugs (1) zum Erzeugen von Strahlung im UV-Bereich aufweist,
- wobei die erste Lichtquelle (3) zum Erzeugen von Strahlung im UV -B- und/oder UV-C-Bereich ausgelegt ist,
- wobei das Desinfektionssystem (2) eine zweite Lichtquelle (4) zum Erzeugen von Strahlung im UV-C-Bereich aufweist,
- wobei die erste Lichtquelle (3) zum Erzeugen von Strahlung im Bereich 100-300 nm, vorzugsweise 200-300 nm, ausgebildet ist, und/oder
- wobei die zweite Lichtquelle (4) zum Erzeugen von Strahlung im Bereich 210-230 nm, vorzugsweise 222 nm, ausgebildet ist,
wobei in dem Verfahren (9):
- eine Steuerung (8) des Desinfektionssystems (2) mittels wenigstens einer Identifikationseinheit (7a, 7b), vorzugsweise einer Kamera oder einem Radar, bestimmt, ob sich Fahrgäste in dem Fahrzeug (1) befinden, und
- die Steuerung (8) abhängig davon, ob sich Fahrgäste in dem Fahrzeug (1) befinden, die erste Lichtquelle (3) oder die zweite Lichtquelle (4) einschaltet,
- wobei, wenn sich keine Fahrgäste in dem Fahrzeug (1) befinden, die Steuerung (8) die erste Lichtquelle (3) für wenigstens 6 Sekunden einschaltet, und
- wobei, wenn sich Fahrgäste in dem Fahrzeug (1) befinden, die Steuerung (8) die zweite Lichtquelle (4) für wenigstens 2,5 Sekunden einschaltet.

2. Desinfektionssystem (2) eines Fahrzeugs (1),
- wobei das Desinfektionssystem (2) eine erste Lichtquelle (3) für einen Innenraum (5) des Fahrzeugs (1) zum Erzeugen von Strahlung im UV-Bereich aufweist,
- wobei die erste Lichtquelle (3) zum Erzeugen von Strahlung im UV -B- und/oder UV-C-Bereich ausgelegt ist,
- wobei das Desinfektionssystem (2) eine zweite Lichtquelle (4) zum Erzeugen von Strahlung im UV-C-Bereich aufweist,
- wobei die erste Lichtquelle (3) zum Erzeugen von Strahlung im Bereich 100-300 nm, vorzugsweise 200-300 nm, ausgebildet ist, und/oder
- wobei die zweite Lichtquelle (4) zum Erzeugen von Strahlung im Bereich 210-230 nm, vorzugsweise 222 nm, ausgebildet ist, und
- wobei das Desinfektionssystem (2) eine Steuerung (8) aufweist, die die erste Lichtquelle (3) und die zweite Lichtquelle (4) steuert und
- wobei das Desinfektionssystem (2) wenigstens eine Identifikationseinheit (7a, 7b) zum Identifizieren von Fahrgästen in dem Fahrzeug (1), vorzugsweise eine Kamera oder ein Radar, aufweist,
**dadurch gekennzeichnet,**
- **dass** das Desinfektionssystem (2) zum Ausführen des Verfahrens (9) nach Anspruch 1 ausgelegt ist.

3. Desinfektionssystem nach Anspruch 2,
**dadurch gekennzeichnet,**
**dass** die zweite Lichtquelle (4) zum Erzeugen von Strahlung mit einer Strahlenkonzentration von wenigstens 12 mJ/cm² ausgebildet ist.

4. Desinfektionssystem nach einem der Ansprüche 2 bis 3,
**dadurch gekennzeichnet,**
**dass** die erste Lichtquelle (3) und/oder die zweite Lichtquelle (4) einteilig oder mehrteilig sind.

5. Fahrzeug (1) mit einem Desinfektionssystem (2) nach einem der Ansprüche 2 bis 4, wobei die erste Lichtquelle (3) und die zweite Lichtquelle (4) in einem Innenraum (5) des Fahrzeugs (1) an einem Dach (6) des Fahrzeugs (1) und/oder an einer tragenden Säule des Fahrzeugs (1) und/oder an einer Konsole des Fahrzeugs (1) befestigt sind.

6. Fahrzeug nach Anspruch 5,
**dadurch gekennzeichnet,**
**dass** die erste Lichtquelle (3) und die zweite Lichtquelle (4) nebeneinander angeordnet sind, so dass die Strahlungsausbreitung der ersten Lichtquelle (3) und die Strahlungsausbreitung der zweiten Lichtquelle (4) in dem Innenraum (5) des Fahrzeugs (1) identisch sind.

## Claims

1. Method (9) for controlling a disinfection system (2) of a vehicle (1),
- wherein the disinfection system (2) has a first light source (3) for an interior (5) of the vehicle (1) for generating radiation in the UV range,
- wherein the first light source (3) is designed to generate radiation in the UV-B and/or UV-C range,
- wherein the disinfection system (2) has a second light source (4) for generating radiation in the UV-C range,
- wherein the first light source (3) is designed to generate radiation in the range of 100-300 nm, preferably 200-300 nm, and/or
- wherein the second light source (4) is designed to generate radiation in the range of 210-230 nm, preferably 222 nm,
wherein in the method (9):
- a controller (8) of the disinfection system (2) determines by means of at least one identification unit (7a, 7b), preferably a camera or a radar, whether passengers are in the vehicle (1), and
- the controller (8) switches on the first light source (3) or the second light source (4) depending on whether passengers are in the vehicle (1),
- wherein, if no passengers are in the vehicle (1), the controller (8) switches on the first light source (3) for at least 6 seconds, and
- wherein, if passengers are in the vehicle (1), the controller (8) switches on the second light source (4) for at least 2.5 seconds.

2. Disinfection system (2) of a vehicle (1),
- wherein the disinfection system (2) has a first light source (3) for an interior (5) of the vehicle (1) for generating radiation in the UV range,
- wherein the first light source (3) is designed to generate radiation in the UV-B and/or UV-C range,
- wherein the disinfection system (2) has a second light source (4) for generating radiation in the UV-C range,
- wherein the first light source (3) is designed to generate radiation in the range of 100-300 nm, preferably 200-300 nm, and/or
- wherein the second light source (4) is designed to generate radiation in the range of 210-230 nm, preferably 222 nm, and
- wherein the disinfection system (2) has a controller (8) which controls the first light source (3) and the second light source (4), and
- wherein the disinfection system (2) has at least one identification unit (7a, 7b) for identifying passengers in the vehicle (1), preferably a camera or a radar,
**characterized in that**
- the disinfection system (2) is designed to carry out the method (9) according to claim 1.

3. Disinfection system according to claim 2,
**characterized in that**
the second light source (4) is designed to generate radiation having a radiation concentration of at least 12 mJ/cm².

4. Disinfection system according to any of claims 2 to 3,
**characterized in that**
the first light source (3) and/or the second light source (4) are single-part or multi-part.

5. Vehicle (1) having a disinfection system (2) according to any of claims 2 to 4,
wherein the first light source (3) and the second light source (4) are fastened in an interior (5) of the vehicle (1) to a roof (6) of the vehicle (1) and/or to a carrying column of the vehicle (1) and/or to a console of the vehicle (1).

6. Vehicle according to claim 5,
**characterized in that**
the first light source (3) and the second light source (4) are arranged next to one another, so that the radiation propagation of the first light source (3) and the radiation propagation of the second light source (4) in the interior (5) of the vehicle (1) are identical.

## Revendications

1. Procédé (9) permettant la commande d'un système de désinfection (2) d'un véhicule (1),
- dans lequel le système de désinfection (2) présente une première source de lumière (3) pour un habitacle (5) du véhicule (1), laquelle permet de générer un rayonnement dans la plage UV,
- dans lequel la première source de lumière (3) est configurée pour générer un rayonnement dans la plage UV B et/ou la plage UV C,
- dans lequel le système de désinfection (2) présente une seconde source de lumière (4), laquelle permet de générer un rayonnement dans la plage UV C,
- dans lequel la première source de lumière (3) est configurée pour générer un rayonnement dans la plage allant de 100 à 300 nm, de préférence de 200 à 300 nm, et/ou
- dans lequel la seconde source de lumière (4) est configurée pour générer un rayonnement dans la plage allant de 210 à 230 nm, de préférence de 222 nm,
dans lequel, dans le procédé (9) :
- un dispositif de commande (8) du système de désinfection (2) détermine, à l'aide d'au moins une unité d'identification (7a, 7b), de préférence une caméra ou un radar, si des passagers se trouvent dans le véhicule (1), et
- en fonction de la présence de passagers dans le véhicule (1), le dispositif de commande (8) active la première source de lumière (3) ou la seconde source de lumière (4),
- dans lequel, lorsqu'aucun passager ne se trouve dans le véhicule (1), le dispositif de commande (8) active la première source de lumière (3) pendant au moins 6 secondes, et
- dans lequel, lorsque des passagers se trouvent dans le véhicule (1), le dispositif de commande (8) active la seconde source de lumière (4) pendant au moins 2,5 secondes.

2. Système de désinfection (2) d'un véhicule (1),
- dans lequel le système de désinfection (2) présente une première source de lumière (3) pour un habitacle (5) du véhicule (1), laquelle permet de générer un rayonnement dans la plage UV,
- dans lequel la première source de lumière (3) est configurée pour générer un rayonnement dans la plage UV B et/ou la plage UV C,
- dans lequel le système de désinfection (2) présente une seconde source de lumière (4), laquelle permet de générer un rayonnement dans la plage UV C,
- dans lequel la première source de lumière (3) est configurée pour générer un rayonnement dans la plage allant de 100 à 300 nm, de préférence de 200 à 300 nm, et/ou
- dans lequel la seconde source de lumière (4) est configurée pour générer un rayonnement dans la plage allant de 210 à 230 nm, de préférence de 222 nm, et
- dans lequel le système de désinfection (2) présente un dispositif de commande (8) qui commande la première source de lumière (3) et la seconde source de lumière (4) et
- dans lequel le système de désinfection (2) présente au moins une unité d'identification (7a, 7b) permettant d'identifier des passagers dans le véhicule (1), de préférence une caméra ou un radar,
**caractérisé en ce que**
- le système de désinfection (2) est configuré pour mettre en oeuvre le procédé (9) selon la revendication 1.

3. Système de désinfection selon la revendication 2,
**caractérisé en ce que**
la seconde source de lumière (4) est configurée pour générer un rayonnement ayant une concentration de rayonnement d'au moins 12 mJ/cm².

4. Système de désinfection selon l'une des revendications 2 à 3, **caractérisé en ce que**
la première source de lumière (3) et/ou la seconde source de lumière (4) sont réalisées en une seule pièce ou en plusieurs pièces.

5. Véhicule (1) comportant un système de désinfection (2) selon l'une des revendications 2 à 4,
dans lequel la première source de lumière (3) et la seconde source de lumière (4) sont fixées dans un habitacle (5) du véhicule (1), à un toit (6) du véhicule (1) et/ou à une colonne porteuse du véhicule (1) et/ou à une console du véhicule (1).

6. Véhicule selon la revendication 5,
**caractérisé en ce que**
la première source de lumière (3) et la seconde source de lumière (4) sont disposées côte à côte, de sorte que la diffusion de rayonnement de la première source de lumière (3) et la diffusion de rayonnement de la seconde source de lumière (4) dans l'habitacle (5) du véhicule (1) sont identiques.
